# EUROPÄISCHE PATENTSCHRIFT

(11) **EP 0 920 275 B1**
(45) Veröffentlichungstag und Bekanntmachung des Hinweises auf die Patenterteilung: **09.10.2002**
(21) Anmeldenummer: 97936571.5
(22) Anmeldetag: 29.07.1997
(51) Int. Cl.: A61B 1/00

(54) **ENDOSKOP MIT WENIGSTENS EINER ERFASSUNGS- UND REGISTRIEREINRICHTUNG**
ENDOSCOPE WITH AT LEAST ONE SENSING AND RECORDING DEVICE
ENDOSCOPE AVEC AU MOINS UN DISPOSITIF DE DETECTION ET D'ENREGISTREMENT

(30) Priorität: 29.07.1996 DE 19630635; 04.06.1997 DE 19723442
(43) Veröffentlichungstag der Anmeldung: 09.06.1999
(73) Patentinhaber: Karl Storz GmbH & Co. KG, 78532 Tuttlingen (DE)
(72) Erfinder: IRION, Klaus, D-78576 Emmingen-Liptingen (DE); RUDISCHHAUSER, Jürgen, D-78532 Tuttlingen (DE)
(74) Vertreter: Münich, Wilhelm, Dr.
(86) Internationale Anmeldenummer: DE9701604
(87) Internationale Veröffentlichungsnummer: WO98004185

(56) Entgegenhaltungen:
- EP-A- 0 561 228
- WO-A-90/04358
- WO-A-94/14129
- DE-A- 3 707 787
- DE-A- 3 741 879
- DE-A- 19 522 909
- US-A- 5 337 732
- US-A- 5 421 821
- US-A- 5 571 133

## Beschreibung

### Technisches Gebiet

Die Erfindung bezieht sich auf ein Endoskop mit einem distal angeordneten Objektiv und einem Bildübertragungssystem, das das Bild des Objektivs zum proximalen Ende überträgt.

### Stand der Technik

Endoskope dieser Art sind allgemein bekannt und werden beispielsweise in der Medizin oder der Technik zur Beobachtung von Hohlräumen insbesondere unter erschwerten Bedingungen eingesetzt.

Das Bildübertragungsystem kann dabei aus einem oder mehreren distal angeordneten Halbleiterchips, wie CCD-Elementen, einem bevorzugt Stablinsen aufweisenden Relaislinsensystem oder einem Faserbündel bestehen.

Die Endoskope werden im Einsatz und/oder bei der Reinigung bzw. Sterilisierung häufig erhöhten Temperaturen - bei der Sterilisierung im Autoklaven bis zu 140°C -, Drücken und/ oder aggressiven Medien - z.B. bei der Sterilisierung in Lösung - ausgesetzt. Dabei sind die Endoskope je nach Bauart nur bis zu bestimmten Umgebungsbedingungen spezifiziert. Werden diese Grenzwerte überschritten, kann es zu den unterschiedlichsten Beschädigungen kommen:
Bei einer falschen Handhabung können Temperaturen und/ oder Drücke auftreten, die über den zulässigen Grenzwerten liegen. Hierdurch können Verbindungsstellen, wie beispielsweise die Verbindung zwischen distalem Endfenster des Objektivs und Außenrohr beschädigt werden, so dass das Endoskop nicht mehr fluiddicht ist, und Feuchtigkeit in den Innenraum eindringen kann. Bei Endoskopen mit einem Halbleiterchip kann dieser durch zu hohe Temperaturen zerstört werden. Ähnliches gilt, wenn das Endoskop anderen Umgebungsbedingungen, für die es nicht spezifiziert ist, und/oder aggressiven Medien ausgesetzt wird.

Zudem können gerade die länglichen Einführteile der Endoskope bei einer unvorsichtigen Handhabung Stößen oder Schlägen ausgesetzt werden. Hierdurch können beispielsweise Stablinsen leicht beschädigt werden und insbesondere brechen. Auch wenn keine Linsen brechen, kann das Endoskop dejustiert werden.

Häufig bemerkt die Bedienungsperson nicht einmal, dass das Endoskop außerhalb der spezifizierten Bedingungen eingesetzt wird. Dies führt nicht nur zu ungerechtfertigten Beanstandungen gegenüber dem Endoskop-Hersteller, sondern auch dazu, dass das Endoskop während eines Einsatzes unvermittelt ausfällt bzw. anderweitige Schäden hervorruft.

Gemäß der WO 94/14129 und DE 39 09 090 C2 wird jeweils insbesondere ein Endoskop mit einem distal angeordneten Objektiv und einem Bildübertragungssystem, das das Bild des Objektivs zum proximalen Ende überträgt, und mit wenigstens einer im Endoskopinnenraum angeordnete Erfassungs- und Registriereinrichtung (4, 5) offenbart, die derart ausgestaltet ist, dass die registrierten bzw. gespeicherten Informationen ohne Demontage des Enkoskops von außen sichtbar, abfragbar und/oder nach außen übertragbar sind.

Gemäß der WO 94/14129 werden zur Erfassung des Nutzungsverlaufs beispielsweise eines Endoskops lediglich die Anzahl von Betätigungsvorgängen bzw. die entsprechende Zeitdauer oder die Anzahl von Sterilisationsvorgängen registriert. Damit können über die Anzahl von Betätigungs- bzw. Sterilisationsvorgängen ungünstige Betriebsbedingungen nur indirekt registriert werden. Es wird lediglich die Anzahl bzw. die Dauer von spezifischen Vorgängen quantitativ ermittelt. Von weiteren Parametern eines Vorgangs, wie beispielsweise die bei einem Vorgang erreichte Temperatur, werden keine Werte registriert. Damit ist die Erfassung des Nutzungsverlaufs ungenügend.

Gemäß der DE 39 09 090 C2 wird zur Erfassung ungünstiger Betriebs- und Umgebungsbedingungen eines Endoskops lediglich die eindringende Luftfeuchtigkeit registriert. Damit ist die Erfassung des Nützungsverlaufs auch hier ungenügend, da anhand der Erfassung dieses einen Parameters, andere wichtige Betriebs- und Umgebungsparamter eines Endoskops, wie z.B. die Temperatur und der Druck, nicht registriert und zur Beurteilung des Zustandes eines Endoskops herangezogen werden. Dies hat zur Folge, dass kritische Situationen nur ungenügend erfassbar sind.

### Darstellung der Erfindung

Der Erfindung liegt die Aufgabe zugrunde, ein gattungsgemäßes Endoskop derart weiterzubilden, dass insbesondere Betriebsbedingungen außerhalb der spezifizierten Bedingungen erkannt werden können.

Eine erfindungsgemäße Lösung dieser Aufgabe ist im Patentanspruch 1 angegeben. Weiterbildungen der Erfindung sind Gegenstand der Ansprüche 2 folgende.

Erfindungsgemäß weist das Endoskop wenigstens eine in das Endoskop integrierte, d.h. im Innenraum des Endoskops angeordnete Erfassungs- und Registriereinrichtung auf, die erfaßt, welchen Temperaturen, Druck, Strahlung, Umgebungsbedingungen und/oder Stoß- bzw. Schlagbelastungen das Endoskop ausgesetzt wird.

Die erfindungsgemäß vorgesehene und in das Endoskop integrierte Erfassungs- und Registriereinrichtung ermöglicht es damit, die "Vorgeschichte" dieses Endoskops zu erfassen, so dass die Ursachen für Ausfälle etc. rekonstruiert werden können.

Weiterhin sind die von der Erfassungs- und Registriereinrichtung registrierten bzw. gespeicherten Informationen, die die "Vorgeschichte" betreffen, ohne Demontage des Endoskops von außen abfragbar. Damit kann eine Bedienungsperson jederzeit erkennen, ob das Endoskop "in der Vergangenheit" unzulässigen Umgebungsbedingungen ausgesetzt worden ist. Dies ermöglicht es der Bedienungsperson insbesondere, ein Endoskop, bei dem aufgrund der Vorgeschichte die Wahrscheinlichkeit eines Ausfalls groß ist, auszusondern, bevor es - beispielsweise während einer Operation - zu dem Ausfall des Endoskops kommt.

Die erfindungsgemäß vorgesehene und ausgebildete Erfassungs- und Registriereinrichtung hat damit eine Kontrollfunktion, die eine Überwachung des Endoskops erlaubt.

Die Erfassungs- und Registriereinrichtung ist dabei so ausgebildet, dass sie den jeweils maximal auftretenden Wert erfasst und speichert sowie zusätzlich die Anzahl kritischer Situationen bzw. Zustände zählt bzw. speichert.

Unter kritischen Situationen bzw. Zuständen werden dabei Zustände verstanden, in denen die für die einzelnen Parameter, wie Temperatur, Druck etc. zulässigen Maximalwerte überschritten werden.

Als Erfassungs- und Registriereinrichtung können im Prinzip beliebige Einrichtungen verwendet werden, die beispielsweise mechanische Indikatoren und/oder elektronische Sensoren aufweisen, solange diese Einrichtungen nur in der Lage sind, die auftretenden Werte der jeweiligen Umgebungsbedingung zu erfassen und zumindest den Spitzenwert zu speichern.

Das Abfragen von außen kann dabei entweder dadurch erfolgen, dass die Erfassungs- und Registriereinrichtung durch ein Fenster im Endoskop sichtbar ist (Anspruch 7) oder die gespeicherten Informationen beispielsweise auf elektrischen Wege und insbesondere berührungslos nach außen übertragen (Anspruch 2 sowie Ansprüche 9 ff) werden.

Eine besonders einfache Ausbildung der Erfassungsund Registriereinrichtung ist im Anspruch 3 angegeben, gemäß der die Erfassungs- und Registriereinrichtung zur Erfassung der auftretenden Temperatur ein Farbindikatorelement aufweist, dessen Farbe den Temperaturwert angibt. Die Farbänderung ist bevorzugt irreversibel, so daß beispielsweise die Maximaltemperatur, der das Endoskop ausgesetzt gewesen ist, zuverlässig auch noch nachträglich festgestellt werden kann.

Im Anspruch 4 ist eine Möglichkeit für eine Erfassungs- und Registriereinrichtung zur Erfassung des auftretenden Drucks angegeben. Die Erfassungs- und Registriereinrichtung weist eine Membran auf, die einen vom eigentlichen Innenraum des Endoskops abgetrennten kleinen Raum, in dem sich Luft mit Normaldruck befindet, abschließt, und die bei einer bestimmten Druckdifferenz zwischen der Umgebung und dem kleinen Raum bricht.

Die im Anspruch 5 gekennzeichnete Erfassungs- und Registriereinrichtung zur Erfassung von Stoß- bzw. Schlagbelastungen weist ein Sollbruchelement auf, das bei einer bestimmten Belastung bricht.

Da es - wie bereits ausgeführt - bevorzugt ist, wenn die von der Erfassungs- und Registriereinrichtung registrierten bzw. gespeicherten Informationen ohne Demontage des Endoskops von außen abfragbar sind, ist es gemäß Anspruch 6 bevorzugt, wenn wenigstens einer der Indikatoren der Erfassungs- und Registriereinrichtung von außen sichtbar ist. Hierzu können der oder die Indikatoren der Erfassungs- und Registriereinrichtung in dem Endoskop hinter einem Fenster und damit von außen sichtbar angeordnet sein (Anspruch 7). Dieses Fenster ist gemäß Anspruch 8 bevorzugt das Okularfenster des Endoskops, so dass es nicht erforderlich ist, ein zusätzliches Fenster in die Außenwand des Endoskops einzubringen.

Wie ebenfalls bereits ausgeführt, können neben den vorstehend genannten mechanisch oder durch Farbumschlag arbeitenden Indikatoren auch elektronische Sensoren verwendet werden.

Anstelle oder zusätzlich zu den vorstehend genannten Indikatorelementen, die durch Farbumschlag oder mechanisch die Überschreitung eines Grenzwertes anzeigen, ist es selbstverständlich auch möglich, daß die Erfassungs- und Registriereinrichtung wenigstens einen Sensor aufweist, der elektronisch erfaßt, welcher Temperatur, welchem Druck, welcher Strahlung bzw. welchen anderen Umgebungsbedingungen oder Stoß- bzw. Schlagbelastungen das jeweilige Endoskop ausgesetzt worden ist. Hierzu weist das Endoskop einen Energiespeicher, einen Sensor, der einen dieser Parameter erfaßt und in ein elektrisches Signal umwandelt, sowie eine Signalverarbeitungseinheit auf, die eine Vorverarbeitung der Parameter ausführt und insbesondere das Überschreiten von Parametern speichern kann. Ferner ist eine Übertragungseinheit vorgesehen, die die gespeicherte Information nach außen überträgt (Anspruch 9).

Dabei ist es weiter bevorzugt, wenn die Erfassungs- und Registriereinrichtung zusätzlich ein Zeiterfassungssystem aufweist, so dass zu den erfaßten Parametern zusätzlich die Zeit, zu der ein kritischer Zustand aufgetreten ist, erfasst und ausgegeben wird. Damit ist es beispielsweise möglich, unsachgemäße Behandlungen eines Endoskops einem bestimmten Benutzer zuzuordnen (Anspruch 10) .

Diese Übertragungseinheit kann ein Stecker, bevorzugt aber ein drahtloses System sein, das insbesondere in Art eines Telemetriesystems ausgebildet sein kann (Anspruch 11).

Ferner ist es bevorzugt, wenn auch der Energiespeicher nach Anspruch 13 von außen - über einen Stecker oder wiederum bevorzugt berührungslos - ladbar ist, da es dann nicht erforderlich ist, das Endoskop zur Wartung der Erfassungs- und Registriereinrichtung zu öffnen.

Wenn die Erfassungs- und Registriereinrichtung wie vorstehend dargelegt als elektronisch arbeitende Erfassungs- und Registriereinrichtung ausgebildet ist, ist es gemäß Anspruch 12 weiterhin bevorzugt, wenn die Erfassungs- und Registriereinrichtung bei Überschreiten bestimmter Schwellwerte für die Temperatur und den Druck weitere Ereigniserkennungen erst nach Ablauf einer bestimmten vorgegebenen Zeit vornimmt. Damit ist es insbesondere möglich, dass die Erfassungs- und Registriereinrichtung die Zahl der Sterilisationszyklen erfaßt, wobei die Sterilisationszyklen entsprechend ihrer jeweiligen Dauer gewichtet werden.

Weiterhin ist es von Vorteil, wenn die Empfangseinrichtung so ausgebildet ist, daß sie die Signale von einer Mehrzahl von Erfassungs- und Registriereinrichtungen empfängt. Hierzu überträgt jede Erfassungs- und Registriereinrichtung zusammen mit den gespeicherten Daten eine eindeutige Identifikation des jeweiligen Endoskops, die insbesondere aus einer Seriennummer des Endoskops, dem Typ des Endoskops und dem Herstelldatum bestehen kann.

Die Empfangseinrichtung speichert die einzelnen Parameter in Zuordnung zur Systemidentifikation, so daß es möglich ist, die einzelnen Werte und insbesondere Parameter-Überschreitungen für jedes Endoskop getrennt abzurufen (Ansprüche 14 bis 16).

Bei der im Anspruch 17 angegebenen Weiterbildung der Erfindung erfaßt bzw. registriert die Erfassungs- und Registriereinrichtung zusätzlich auch Funktionsparameter des Endoskops, wie beispielsweise die Stellung eines Zoom- bzw. Varioobjektives, die Stellung eines endoskopinternen Erfassungs- und Registriersystems usw.

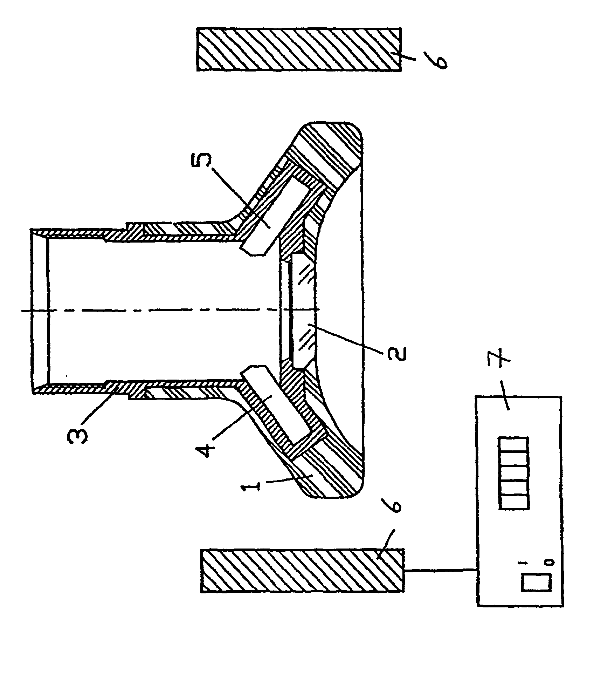

### Kurze Beschreibung der Zeichnung

Die Erfindung wird nachstehend anhand eines Ausführungsbeispiels unter Bezugnahme auf die Zeichnung beschrieben, die
einen Längsschnitt durch den proximalen Teil eines Endoskops und eine Empfangseinrichtung
zeigt.

### Darstellung eines Ausführungsbeispiels

In der Zeichnung ist der proximale Teil eines Endoskops, nämlich eine Okularmuschel 1 mit einem Okularfenster 2 dargestellt. An die Okularmuschel 1 schließt sich der proximale Teil des Endoskops an, an dem sich der in der Zeichnung nicht dargestellte Lichtleiteranschluß befindet.

Im Bereich der Okularmuschel 1 sind Elemente 4 und 5 einer Erfassungs- und Registriereinrichtung dargestellt.

Bei dem Element 4 handelt es sich um ein Indikatorelement, das beispielsweise die maximal im Innenraum des Endoskops auftretende Temperatur durch einen Farbumschlag angibt. Das Element 4 kann dabei durch das Okularfenster 2 betrachtet werden.

Bei dem Element 5 handelt es sich ohne Beschränkung der Allgemeinheit um eine elektronische Sensoreinrichtung, die einen von außen nach dem Prinzip der Telemetrie ladbaren Energiespeicher, einen Sensor, der einen kritischen Parameter, wie Temperatur, Druck, Strahlung, Beschleunigung etc. erfaßt und in ein elektrisches Signal wandelt sowie eine Signal-Vorverarbeitungseinheit aufweist.

Ferner ist als Übertragungseinrichtung ein Sendeelement integriert, das ebenfalls nach dem Prinzip der Telemetrie - Modulation eines von außen eingestrahlten Wellenfeldes durch die Übertragungseinheit zur Minimierung der für die Übertragung erforderlichen Leistung - arbeiten kann.

Mit 6 ist eine Antenne für das abgestrahlte Signal bezeichnet. Das Ausgangssignal der Antenne 6 wird an eine elektronische Empfangs- und Auswerteeinrichtung 7 angelegt, das aus dem von der Sensoreinheit 5 abgegebenen Signal und einer eindeutigen Systemidentifikation für das jeweilige Endoskop besteht. Die Systemidentifikation kann insbesondere aus einer Seriennummer des Endoskops, dem Typ des Endoskops und dem Herstelldatum bestehen. Die Empfangs- und Auswerteeinrichtung speichert die einzelnen Parameter in Zuordnung zur Systemidentifikation, so daß es möglich ist, die einzelnen Werte und insbesondere Parameter-Überschreitungen für jedes Endoskop getrennt abzurufen.

## Patentansprüche

1. Endoskop mit einem distal angeordneten Objektiv und
einem Bildübertragungssystem, das das Bild des Objektivs zum proximalen Ende überträgt,
wenigstens einer im Endoskopinnenraum angeordnete Erfassungs- und Registriereinrichtung (4, 5), die derart ausgestaltet ist, dass die registrierten bzw. gespeicherten Informationen ohne Demontage des Enkoskops von außen sichtbar, abfragbar und/oder nach außen übertragbar sind,
**dadurch gekennzeichnet, dass** die Erfassungs- und Registriereinrichtung die Temperatur, den Druck, die Strahlung und/oder Stoß- bzw. Schlagbelastungen erfaßt, denen das Endoskop ausgesetzt wird, und
dass die Erfassungs- und Registriereinrichtung den jeweils maximal auftretenden Wert erfasst, wobei die Erfassungs- und Registriereinrichtung die Anzahl kritischer Situationen, in denen zulässige Maximalwerte überschritten werden, zählt und speichert.

2. Endoskop nach Anspruch 1,
**dadurch gekennzeichnet, dass** ein Übertragungssystem vorgesehen ist, das die von der Erfassungsund Registriereinrichtung registrierten bzw. gespeicherten Informationen berührungslos in den Umgebungsraum des Endoskops überträgt.

3. Endoskop nach einem der Ansprüche 1 oder 2,
**dadurch gekennzeichnet, dass** die Erfassungs- und Registriereinrichtung zur Erfassung der auftretenden Temperatur ein Farbindikatorelement aufweist, dessen Farbe den Temperaturwert angibt.

4. Endoskop nach einem der Ansprüche 1 bis 3,
**dadurch gekennzeichnet, dass** die Erfassungs- und Registriereinrichtung zur Erfassung des auftretenden Drucks eine Membran als Indikator aufweist, die bei einer bestimmten Druckdifferenz bricht.

5. Endoskop nach einem der Ansprüche 1 bis 4,
**dadurch gekennzeichnet, dass** die Erfassungs- und Registriereinrichtung zur Erfassung von Stoß- bzw. Schlagbelastungen als Indikator ein Sollbruchelement ist, das bei einer bestimmten Belastung bricht.

6. Endoskop nach einem der Ansprüche 3 bis 5,
**dadurch gekennzeichnet, dass** wenigstens einer der Indikatoren der Erfassungs- und Registriereinrichtung von außen sichtbar ist.

7. Endoskop nach Anspruch 6,
**dadurch gekennzeichnet, dass** der oder die Indikatoren der Erfassungs- und Registriereinrichtung in dem Endoskop hinter einem Fenster und damit von außen sichtbar angeordnet sind.

8. Endoskop nach Anspruch 7,
**dadurch gekennzeichnet, dass** das Fenster das Okularfenster ist.

9. Endoskop nach einem der Ansprüche 1 bis 8,
**dadurch gekennzeichnet, dass** die Erfassungs- und Registriereinrichtung wenigstens einen elektrischen Energiespeicher, einen Sensor, der die auf das Endoskop einwirkende Temperatur, den Druck, die Strahlung, die Beschleunigung in ein elektrisches Signal umwandelt, eine Signal-Vorverarbeitungseinheit und eine Signal- Übertragungseinrichtung aufweist, die das vorverarbeitete Signal nach außen zu einer Empfangseinrichtung überträgt.

10. Endoskop nach Anspruch 9,
**dadurch gekennzeichnet, dass** die Erfassungs- und Registriereinrichtung zusätzlich ein Zeiterfassungssystem aufweist, so dass zu den erfassten Parametern zusätzlich die Zeit, zu der ein kritischer Zustand aufgetreten ist, erfasst und ausgegeben wird.

11. Endoskop nach Anspruch 9 oder 10,
**dadurch gekennzeichnet, dass** die Übertragungseinrichtung zu drahtlosen Übertragung der Informationen als Sendeeinrichtung ausgebildet ist.

12. Endoskop nach Anspruch 11,
**dadurch gekennzeichnet, dass** die Signal-Vorverarbeitungseinheit bei Überschreiten von Schwellwerten für die Temperatur und/oder den Druck eine weitere Ereigniserkennung erst nach Ablauf einer vorgegebenen Zeit vornimmt.

13. Endoskop nach einem der Ansprüche 9 bis 12,
**dadurch gekennzeichnet, dass** der Energiespeicher von außen ladbar ist.

14. Endoskop nach einem der Ansprüche 9 bis 13,
**dadurch gekennzeichnet, dass** die Erfassungs- und Registriereinrichtung zusammen mit den gespeicherten Daten eine eindeutige Identifikation des jeweiligen Endoskops überträgt, die insbesondere aus einer Seriennummer des Endoskops, dem Typ des Endoskops und dem Herstelldatum bestehen kann.

15. Endoskop nach Anspruch 14,
**dadurch gekennzeichnet, dass** die Empfangseinrichtung die einzelnen Parameter in Zuordnung zur Systemidentifikation speichert.

16. Endoskop nach Anspruch 14 oder 15,
**dadurch gekennzeichnet, dass** die die Empfangseinrichtung so ausgebildet ist, dass sie die Signale von einer Mehrzahl von Erfassungs- und Registriereinrichtungen empfängt.

17. Endoskop nach einem der Ansprüche 1 bis 16,
**dadurch gekennzeichnet, dass** die Erfassungs- und Registriereinrichtung zusätzlich auch Funktionsparameter des Endoskops registriert.

## Claims

1. Endoscope comprising a distally disposed objective and
an image transmission system that transmits the image from the objective to the proximal end,
at least one sensing and recording means (4, 5) disposed in the inner space of the endoscope, which means is so designed that the recorded or stored information is visible or retrievable from the outside and/or may be transmitted to the outside without disassembly of the endoscope,
**characterised in that** said sensing and recording means detects the temperature, the pressure, the radiation and/or shock or impact loads, respectively, which the endoscope is exposed to, and
that said sensing and recording means detects the respective maximum value occurring, with said sensing and recording means counting and storing the number of critical situations in which admissible maximum values are exceeded.

2. Endoscope according to Claim 1,
**characterised in that** a transmission system is provided that transmits the information detected or stored by said sensing and recording means, without contact into the space surrounding the endoscope.

3. Endoscope according to any of the Claims 1 or 2,
**characterised in that** said sensing and recording means comprises a colour indicator element for detecting the occurring temperature, whose colour reflects the temperature value.

4. Endoscope according to any of the Claims 1 to 3,
**characterised in that** said sensing and recording means comprises a membrane as indicator for the detection of the occurring pressure, which breaks at a defined pressure difference.

5. Endoscope according to any of the Claims 1 to 4,
**characterised in that** said sensing and recording means for the detection of shock or impact loads, respectively, is a rated break element as indicator, which breaks at a defined load.

6. Endoscope according to any of the Claims 3 to 5,
**characterised in that** at least one of said indicators of said sensing and recording means is visible from the outside.

7. Endoscope according to Claim 6,
**characterised in that** said indicator or indicators of said sensing and recording means is/are disposed behind a window and hence for visibility from the outside.

8. Endoscope according to Claim 7,
**characterised in that** said window is the eyepiece window.

9. Endoscope according to any of the Claims 1 to 8,
**characterised in that** said sensing and recording means comprises at least one electrical energy accumulator, a sensor converting the temperature, the pressure, the radiation, the acceleration acting upon the endoscope into an electrical signal, a signal pre-processing unit and a signal transmission means that transmits the pre-processed signal to the outside to a receiving means.

10. Endoscope according to Claim 9,
**characterised in that** said sensing and recording means comprises additionally a time detection system so that in addition to the sensed parameters also the time is detected and output by which a critical state has occurred.

11. Endoscope according to Claim 9 or 10,
**characterised in that** said transmission means is configured as transmitter means for wireless transmission of the information.

12. Endoscope according to Claim 11,
**characterised in that** when thresholds for the temperature and/or the pressure are exceeded said signal pre-processing unit performs a further recognition of an event only upon expiration of a predetermined interval.

13. Endoscope according to any of the Claims 9 to 12,
**characterised in that** said energy accumulator is adapted to be charged from the outside.

14. Endoscope according to any of the Claims 9 to 13,
**characterised in that** said sensing and recording means transmits, together with the stored data, an unambiguous identifier of the respective endoscope, which may consist in particular of a serial number of the endoscope, the type of the endoscope and the manufacturing date.

15. Endoscope according to Claim 14,
**characterised in that** said receiving means stores the individual parameters, associating them with the system identifier.

16. Endoscope according to Claim 14 or 15,
**characterised in that** said receiving means is so configured that it receives the signals from a plurality of sensing and recording means.

17. Endoscope according to any of the Claims 1 to 16,
**characterised in that** said sensing and recording means records additionally also functional parameters of the endoscope.

## Revendications

1. Endoscope comprenant un objectif disposé du côté distal, et
un système de transmission d'images, qui transmet l'image de l'objectif à l'extrémité proximale,
au moins one moyen détecteur enregistreur (4, 5) disposé dans l'espace intérieur de l'endoscope, ce moyen étant conçu de façon, que les informations enregistrées ou mises en mémoire soient visibles ou lisibles de l'extérieur et/ou puissent être transmises à l'extérieur sans démontage de l'endoscope,
**caractérisé en ce que** ledit moyen détecteur enregistreur détecte la température, la pression, le rayonnement et/ou les charges par choc ou respectivement par impact, auxquels l'endoscope est exposé, et
**en ce que** ledit moyen détecteur enregistreur détecte la valeur maximale respectivement présente, audit moyen détecteur enregistreur comptant et mettant en mémoire le nombre de situations critiques, dans lesquelles des valeurs maximales admissibles sont dépassées.

2. Endoscope selon la revendication 1,
**caractérisé en ce qu'**un système de transmission system est disposé, qui transmet les informations détectées ou mises en mémoire par ledit moyen détecteur enregistreur, sans contact dans l'espace autour de l'endoscope.

3. Endoscope selon une quelconque des revendications 1 ou 2,
**caractérisé en ce que** ledit moyen détecteur enregistreur comprend un élément indicateur en couleur à détecter la température régnante, dont la couleur indique la valeur de la température.

4. Endoscope selon une quelconque des revendications 1 à 3,
**caractérisé en ce que** ledit moyen détecteur enregistreur comprend une membrane comme l'indicateur pour la détection de la pression respectivement régnante, qui rompe à une différence en pression définie.

5. Endoscope selon une quelconque des revendications 1 à 4,
**caractérisé en ce que** ledit moyen détecteur enregistreur pour la détection des charges par choc ou respectivement par impact est un élément destiné à la rupture pour la fonction d'indicateur, qui rompe à une charge définie.

6. Endoscope selon une quelconque des revendications 3 à 5,
**caractérisé en ce qu'**au moins un desdits indicateurs dudit moyen détecteur enregistreur est visible de l'extérieur.

7. Endoscope selon la revendication 6,
**caractérisé en ce que** ledit indicateur ou lesdits indicateurs dudit moyen détecteur enregistreur est/sont disposé(s) derrière une fenêtre et est/sont visible de l'extérieur.

8. Endoscope selon la revendication 7,
**caractérisé en ce que** ladite fenêtre est la fenêtre dans l'oculaire.

9. Endoscope selon une quelconque des revendications 1 à 8,
**caractérisé en ce que** ledit moyen détecteur enregistreur comprend au moins un accumulateur d'énergie électrique, un détecteur à convertir la température, la pression, le rayonnement, l'accélération agissant sur l'endoscope en un signal électrique, une unité de prétraitement des signaux et un moyen de transmission des signaux, qui transmet le signal prétraité à l'extérieur moyennant un moyen récepteur.

10. Endoscope selon la revendication 9,
**caractérisé en ce que** ledit moyen détecteur enregistreur comprend au plus un système détecteur du temps de façon, qu'au plus aux paramètres détectés le temps est également détecté et sorti, auquel un état critique apparaissait.

11. Endoscope selon la revendication 9 ou 10,
**caractérisé en ce que** ledit moyen de transmission est configuré sous forme d'un émetteur pour la transmission des informations sans fil.

12. Endoscope selon la revendication 11,
**caractérisé en ce que** quand des seuils pour la température et/ou la pression sont dépassés, ladite unité de prétraitement des signaux ne réalise une opération additionnelle de reconnaissance d'un évènement qu'après la fin d'un intervalle prédéterminé.

13. Endoscope selon une quelconque des revendications 9 à 12,
**caractérisé en ce que** ledit accumulateur d'énergie est adapté à être chargé de l'extérieur.

14. Endoscope selon une quelconque des revendications 9 à 13,
**caractérisé en ce que** ledit moyen détecteur enregistreur transmet, ensemble avec les données mémorisées, un identificateur univoque de l'endoscope respectif, qui peut être composé, en particulier, d'un numéro de série de l'endoscope, le modèle de l'endoscope et la date de fabrication.

15. Endoscope selon la revendication 14,
**caractérisé en ce que** ledit moyen récepteur met en mémoire les paramètre individuels, en les affectant à l'identificateur du système.

16. Endoscope selon la revendication 14 ou 15,
**caractérisé en ce que** ledit moyen récepteur est configuré de façon, qu'il reçoive les signaux d'une pluralité de moyens détecteurs enregistreurs.

17. Endoscope selon une quelconque des revendications 1 à 16,
**caractérisé en ce que** ledit moyen détecteur enregistreur enregistre au plus des paramètres fonctionnels de l'endoscope.
